# EUROPEAN PATENT APPLICATION

(11) **EP 2 957 222 A1**
(43) Date of publication of application: **23.12.2015**
(21) Application number: 15172984.5
(22) Date of filing: 19.06.2015
(51) Int. Cl.: A61B 5/00

(54) **PHOTOACOUSTIC IMAGING DEVICE**

(30) Priority: 20.06.2014 JP 2014127542
(71) Applicant: Funai Electric Co., Ltd., Daito city Osaka 574-0013 (JP)
(72) Inventor: Shimada, Kousuke, Osaka, Osaka 574-0013 (JP)
(74) Representative: Osha Liang

(57) **Abstract**

A photoacoustic imaging device includes a connector (11) that connects a light source device (20), a booster circuit (13) that includes a capacitor (13d) and that performs a boosting operation that applies voltage to the light source device (20) via the connector (11), a discharge circuit (15) connected to both poles of the capacitor (13d) of the booster circuit (13) and that performs a discharge operation that discharges a charge stored in the capacitor (13d), and a discharge circuit controller (16) that controls the discharge circuit to perform the discharge operation when the connector (11) and the light source device (20) are disconnected or when the power source of the photoacoustic imaging device main body is off.

## Description

### [Technical Field]

This invention generally relates to a photoacoustic imaging device, and more particularly to a photoacoustic imaging device that includes a discharge circuit.

### [Background]

Conventional drive circuit configurations (circuit packages) comprise a discharge circuit (for example, refer to Patent Literature 1).

Patent Literature 1 discloses a circuit package comprising a capacitor, a detection circuit that detects the connection state of the circuit package and a main body device, and a discharge circuit connected to both poles of the capacitor. The circuit package prohibits a discharge operation of the discharge circuit when the detection circuit detects that the circuit package and the main body device are connected. The circuit package discharges a charge stored in the capacitor of the circuit package by operating the discharge operation of the discharge circuit when the detection circuit detects that the circuit package is separated from the main body device.

Meanwhile, conventional photoacoustic imaging devices image information inside of a subject. The conventional photoacoustic imaging devices may comprise a light source device separated from the main body of the photoacoustic imaging device. The conventional photoacoustic imaging devices cause the photoacoustic imaging device main body to supply electric power to the light source device.

### [Citation List]

### [Patent Literature]

[Patent Literature 1] Japanese Unexamined Patent Application Publication No. 1996-205401

However, according to the circuit package disclosed in Patent Literature 1, while the capacitor in the circuit package is not discharged by the discharge circuit of the circuit package, the capacitor in the main body device is not discharged. As a result, the electrical circuit may be damaged due to the charge stored in the capacitor in the main body device.

Furthermore, even if the circuit package of Patent Literature 1 is applied to the photoacoustic imaging device described above, the electrical circuit may be damaged due to the charge stored in the capacitor in the main body of the photoacoustic imaging device.

### [Summary of the Invention]

A photoacoustic imaging device in accordance with one or more embodiments can suppress damage of an electric circuit due to a charge stored in a capacitor in a main body of the photoacoustic imaging device.

A photoacoustic imaging device according to one or more embodiments of the present invention may comprise a connector that connects a light source device, a booster circuit that includes a capacitor and that performs a boosting operation that applies voltage to the light source device via the connector, a discharge circuit connected to both poles of the capacitor of the booster circuit and that performs a discharge operation that discharges a charge stored in the capacitor, and a discharge circuit controller that controls the discharge circuit to perform the discharge operation when the connector and the light source device are disconnected or when the power source of the photoacoustic imaging device main body is off.

A photoacoustic imaging device according to one or more embodiments of the present invention may comprise a connector provided on the photoacoustic imaging device that connects a light source device provided separately from the photoacoustic imaging device main body, a booster circuit provided on the photoacoustic imaging device, including a capacitor, which applies voltage to the light source device via the connector, a discharge circuit provided on the photoacoustic imaging device, connected to both poles of the capacitor of the booster circuit, which discharges a charge stored in the capacitor, and a discharge circuit controller provided on the photoacoustic imaging device, that controls the discharge circuit to perform a discharge operation when the connection of the connector to the light source device is removed or when the power source of the photoacoustic imaging device main body is off.

The photoacoustic imaging device according to one or more embodiments of the present invention, as described above, may comprise a discharge circuit controller that controls the discharge circuit to perform a discharge operation that discharges a charge stored in the capacitor provided on the photoacoustic imaging device main body when the connection between the main body side connector and the light source device is removed and when the power source of the photoacoustic imaging device main body is off. According to this configuration, for example, the discharge circuit may discharge the charge stored in the capacitor provided on the photoacoustic imaging device main body even when the power source is on when the connection between the main body side connector and the light source device is removed. For example, the discharge circuit may rapidly discharge the charge stored in the capacitor provided on the photoacoustic imaging device main body when the power source is off. As a result, for example, damage of an electric circuit may be suppressed due to a charge stored in the capacitor in the photoacoustic imaging device main body when the connection of the connector and the light source device is removed or when the power source of the photoacoustic imaging device main body is off.

According to one or more embodiments of the present invention, the discharge circuit controller may comprise a power source detector that detects whether a power source is on or off, and the connection detector that detects the connection of the light source device to the connector and the disconnection. According to this configuration, for example, the power source detector may be able to simply detect whether a power source is on or off, and the connection detector may be able to simply detect whether there is a connection.

According to one or more embodiments of the present invention, the power source detector may determine that a power source is on when it is detected that the voltage of the power source exceeds a threshold voltage, and may determine that a power source is off when the threshold voltage is not exceeded. According to this configuration, for example it may be easily determined whether a power source is on or off by only detecting that the voltage of the power source has exceeded a threshold voltage.

According to one or more embodiments of the present invention, threshold voltage determined that the power source is on may be different from threshold voltage determined that the power source is off. According to this configuration, for example, the photoacoustic imaging device may prevent malfunction even if noise appears in power source voltage.

According to one or more embodiments of the present invention, the booster circuit may stop a boosting operation while performing the discharge operation. According to this configuration, for example, the photoacoustic imaging device may prevent damage of the electric circuit due to excess current.

According to one or more embodiments of the present invention, the booster circuit may stop the boosting operation during longer than a time constant determined based on a discharge resistance and the capacitor of the photoacoustic imaging device main body. According to this configuration, for example, the photoacoustic imaging device may cause the power source to restart faster because voltage of the capacitor is reduced to less than approximately one third.

According to one or more embodiments of the present invention, the discharge circuit may comprise a switching element that performs a switch of the discharge operation by turning on and off according to a signal output from the discharge circuit controller. According to this configuration, for example, the discharge operation of the discharge circuit can be switched with a simple configuration.

According to one or more embodiments of the present invention, the discharge circuit controller may comprise a discharge resistance. According to this configuration, for example, the photoacoustic imaging device may limit current flowing while the discharge operation is performed.

According to one or more embodiments of the present invention, a light emitting element of the light source device may include a plurality of light emitting elements connected in series. According to this configuration, for example, the photoacoustic imaging device may cause the light source to maintain uniform light intensity because current value flowing through each light emitting element is equal.

According to one or more embodiments of the present invention, the discharge circuit controller may comprise a genuine product detector that detects that either a genuine product or a non-genuine product of the light source device is connected, and that the discharge circuit controller controls the discharge circuit to perform a discharge operation when it has been detected that a non-genuine product is connected by the genuine product detector, in addition to when the connection of the connector and the light source device is removed, and when the power source is off. According to this configuration, for example, the booster circuit may not perform a boosting operation because the discharge circuit may perform a discharge operation when a non-genuine product of the light source device is accidentally connected. Therefore, one or more embodiments of the present invention may suppress to cause voltage boosted by the booster circuit to be applied to a non-genuine product even if a non-genuine product of the light source device is accidentally connected.

According to one or more embodiments of the present invention, the light emitting element of the light source device may be configured by a light emitting diode. According to this configuration, for example, irradiation range of light is not easily changed relatively even when positions of the light emitting element is misaligned because directivity of the light emitting diode is lower than that of a light emitting semiconductor element. As a result, enlargement and complication of the photoacoustic imaging device may be suppressed because precise alignment of optical components, an optical surface plate, and a strong case are not required.

According to one or more embodiments of the present invention, the light emitting element of the light source device may be configured by a semiconductor laser element. According to this configuration, for example, the photoacoustic imaging device may cause the semiconductor laser element to surely irradiate a large part of light to a subject because directivity of the semiconductor laser element is higher than that of a light emitting diode.

According to one or more embodiments of the present invention, the light emitting element of the light source device may be configured by an organic light emitting diode. According to this configuration, for example, the photoacoustic imaging device may cause the light source device including light emitting semiconductor element to be easily downsized because the organic light emitting diode is easily thinned.

According to one or more embodiments of the present invention, the discharge circuit controller may control the discharge circuit not to perform a discharge operation when it is detected that a genuine product is connected by the genuine product detector, and when the power source is on. According to this configuration, for example, the booster circuit may appropriately perform the boosting operation when a genuine product of the light source device is connected.

The photoacoustic imaging device according to one or more embodiments of the present invention may suppress damage of an electric circuit due to a charge stored in a capacitor in a main body of the photoacoustic imaging device.

### [Brief Description of Drawings]

FIG. 1 is a diagram of the entire configuration of the photoacoustic imaging device according to one or more embodiments of first and second examples of the present invention.
FIG. 2 is a schematic circuit diagram showing a configuration of circuits of the photoacoustic imaging device and a light source device according to one or more embodiments of the first example of the present invention.
FIG. 3 is a diagram for explaining an operation of a discharge circuit when the connection of a main body side connector and the light source device is removed when the power of the photoacoustic imaging device is off according to one or more embodiments of the first example of the present invention.
FIG. 4 is a diagram for describing an operation of the discharge circuit when the connection of a main body side connector and the light source device is removed when the power of the photoacoustic imaging device is on according to one or more embodiments of the first example of the present invention.
FIG. 5 is a diagram showing the photoacoustic imaging device connected to a genuine product of the light source device according to one or more embodiments of the second example of the present invention.
FIG. 6 is a diagram showing the photoacoustic imaging device connected to a non-genuine product of the light source device according to one or more embodiments of the second example of the present invention.
FIG. 7 is a diagram of the entire configuration of the photoacoustic imaging device according to one or more embodiments of a third embodiment of the present invention.
FIG. 8 is a diagram showing an example of the photoacoustic imaging device connected to the genuine product of the light source device according to one or more embodiments of the third example of the present invention.
FIG. 9 is a diagram showing another example of the photoacoustic imaging device connected to the genuine product of the light source device according to one or more embodiments of the third example of the present invention.
FIG. 10 is a diagram showing another example of the photoacoustic imaging device connected to the genuine product of the light source device according to one or more embodiments of the third example of the present invention.
FIG. 11 is a diagram showing the photoacoustic imaging device connected to the non-genuine product of the light source device according to one or more embodiments of the third example of the present invention.

### [Detailed Description of Embodiments]

Embodiments of the present invention will be described below, with reference to the drawings.

### (First Example)

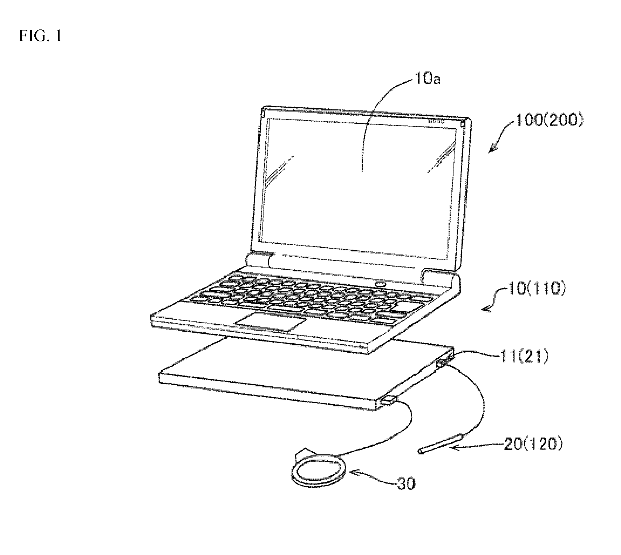

First, the configuration of a photoacoustic imaging device 100 according one or more embodiments of the first example of the present invention will be described with reference to FIG. 1.

The photo acoustic imaging device 100 according to one or more embodiments of the first example of the present invention may comprise a photoacoustic imaging device main body 10 (disclosed as device main body 10 below), a light source device 20, and a probe 30, as illustrated in FIG. 1.

According to one or more embodiments, the photoacoustic imaging device 100 irradiates light from the light source device 20 to a subject (for example, a human body) and detect photoacoustic waves (ultrasound waves) generated from the subject by the probe 30. Then, the photoacoustic imaging device 100 makes information from inside the subject visible (imaging) based on the detection results of the probe 30, and displays on a display unit 10a of the device main body 10.

According to one or more embodiments, in the photoacoustic imaging device 100, the light source device 20 is connected to a main body side connector 11 of the device main body 10. For example, in the photoacoustic imaging device 100, the device main body 10 and the light source device 20 are connected by a light source side connector 21 of the light source device 20 being inserted into the main body side connector 11. Electric power is supplied to the light source device 20 from the main body device 10 with the light source device 20 (light source side connector 21) being connected to the main body side connector 11. As a result, the light source device 20 can irradiate light. The main body side connector 11 is an example of a "connector" of one or more embodiments of the present invention.

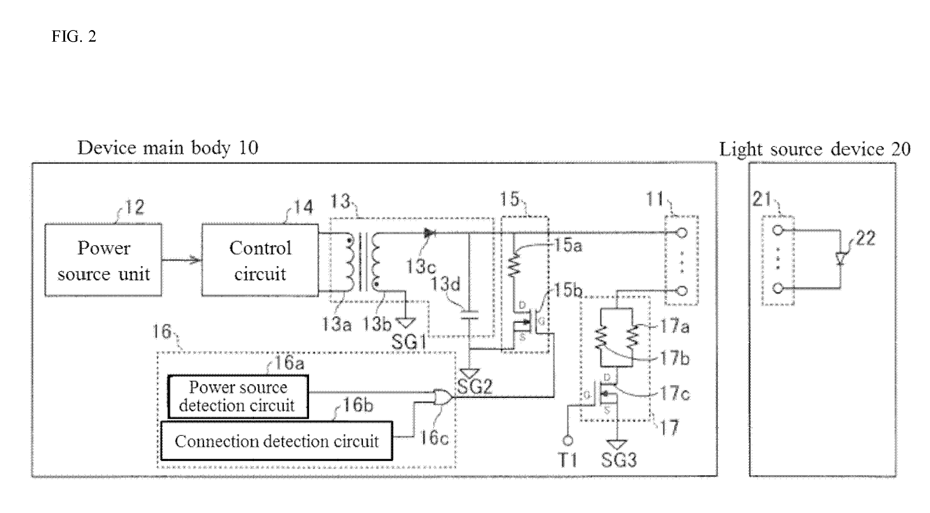

A configuration of circuits of the device main body 10 and the light source device 20 will be described with reference to FIG. 2. For example, the configuration of circuits for supplying electric power to the light source device 20 from the device main body 10 will be described.

According to one or more embodiments, the device main body 10 may comprise a power source unit 12. The power source unit 12 includes a direct current power source such as a battery and supplies electric power (direct current electric power) to each component of the device main body 10. The power source unit 12 may supply direct current electric power by the combining of an alternating current power source such as a commercial power source and a rectification smoothing circuit.

According to one or more embodiments, the device main body 10 may comprise a booster circuit 13. The booster circuit 13 includes a primary coil 13a, a secondary coil 13b, a diode 13c, and a capacitor 13d.

Both ends of the primary coil 13a are connected to a control circuit 14. The control circuit 14 controls by a switching operation of current flowing in the primary coil 13a to generate direct current voltage in the secondary coil 13b.

The secondary coil 13b includes one end connected to a signal ground (SG) 1, and the other end is connected to the diode 13c.

The diode 13c is connected to a terminal of the main body side connector 11 and an electrode of one side of the capacitor 13d.

The capacitor 13d has the electrode of the other side connected to an SG2. The booster circuit 13 includes a rectification smoothing circuit configured by the diode 13c and the capacitor 13d. The direct current voltage generated in the secondary coil 13b of the booster circuit 13 is rectified and smoothed. The direct current voltage boosted to a predetermined voltage (several hundred volts) by the booster circuit is applied to the light source device 20 via the main body side connector 11.

According to one or more embodiments, the device main body 10 may comprise a discharge circuit 15. The discharge circuit 15 includes a discharge resistance 15a and a field effect transistor (FET) 15b.

The discharge resistance 15a includes one end connected to the electrode of one side of the capacitor 13d of the booster circuit 13 and the other end connected to a drain (D) of the FET 15b.

The FET 15b has a source (S) connected to the electrode on the other side of the capacitor 13d of the booster circuit 13. A gate (G) of the FET 15b is connected to an OR circuit 16c of a discharge controller 16 described later. The FET 15b turns on and off according to a signal output from the OR circuit 16c. The FET 15b is one example of a "switching element" of one or more embodiments of the present invention.

The discharge circuit 15 discharges a charge stored in the capacitor 13d. For example, in the discharge circuit 15, the other end of the discharge resistance 15a is electrically connected to the capacitor 13d via the FET 15b when the FET 15b is on. The discharge resistance 15a may limit discharge current because the discharge resistance 15a may determine a peak value of the discharge current. Thus, the charge stored in the capacitor 13d is discharged by the discharge resistance 15a, and the discharge circuit 15 performs the discharge operation based on a time constant determined based on a capacity C of the capacitor 13d and a resistance value R of the discharge resistance 15a. As a result, for example, the photoacoustic imaging device 10 may cause the power source unit 12 to restart faster because voltage of the capacitor 13d is reduced to less than approximately one third.

In the discharge circuit 15, the electrical connection of the other end of the discharge resistance 15a and the capacitor 13d via the FET 15b is removed when the FET 15b is off. As a result, the charge stored in the capacitor 13d is not discharged by a discharge resistance 15a and the discharge operation is not performed. According to one or more embodiments of the present invention, threshold voltage for determining that the power source is on may be different from threshold voltage for determining that the power source is off. According to this configuration, for example, the photoacoustic imaging device 10 may prevent malfunction even if noise appears in power source voltage.

According to one or more embodiments, the device main body 10 may comprise a discharge circuit controller 16. According to one or more embodiments of the first example of the present invention, the discharge circuit controller 16 controls the discharge circuit 15 to perform the discharge operation by turning the FET 15b on, when the connection between the main body side connector 11 and the light source device 20 is removed or when the power source (power source unit 12) of the device main body 10 is off. The discharge circuit controller 16 controls the discharge circuit 15 not to perform the discharge operation by turning the FET 15b off, when the main body side connector 11 and the light source device 20 are connected, and when the power source is on.

According to one or more embodiments, the discharge circuit controller 16 includes a power source detection circuit 16a, a connection detection circuit 16b, and the OR circuit 16c. The power source detection circuit 16a and the connection detection circuit 16b are both connected to an input end of the OR circuit 16c. The output end of the OR circuit 16c is connected to the gate of the FET 15b. The power source detection circuit 16a and the connection detection circuit 16b are each examples of "power source detector" and "connection detector" of one or more embodiments of the present invention.

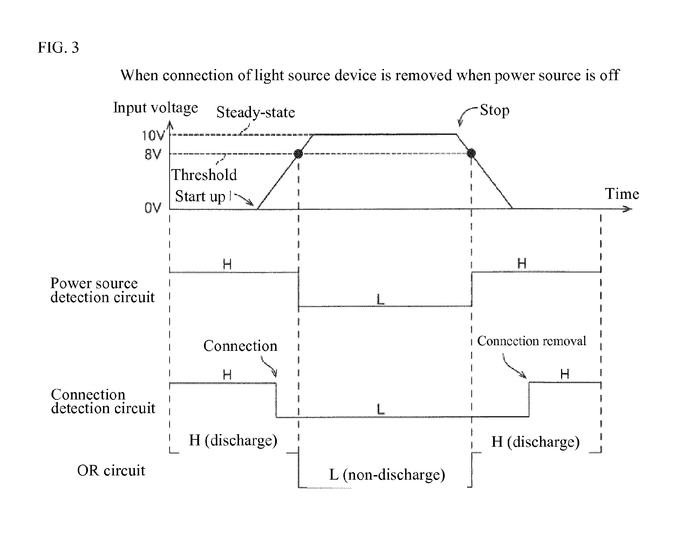

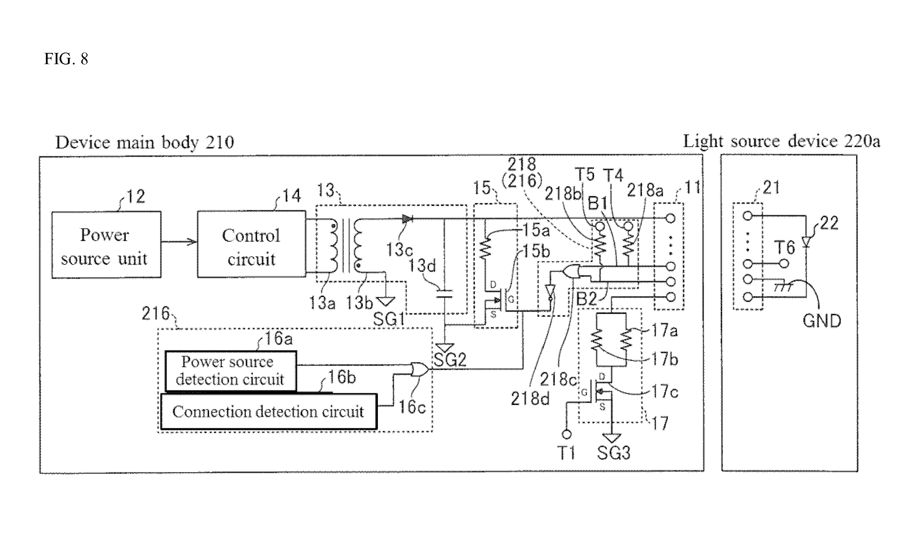

According to one or more embodiments of the first example of the present invention, the power source detection circuit 16a detects whether a power source is on or off by detecting voltage (input voltage) applied to the gate of an FET 17c of a current limiting circuit 17 described below. For example, the power source detection circuit 16a determines (detects) that the power source is on when it is detected that the voltage applied to the gate of the FET 17c of the current limiting circuit 17 exceeds a threshold voltage (refer to FIG. 3, 8V), and determines (detects) that the power source is off when the threshold voltage is not exceeded. Also, the power source detection circuit 16a outputs a low level signal to the input end of the OR circuit 16c when it is detected that the power source is on, and output a high level signal to the input end of the OR circuit 16c when it is determined that the power source is off.

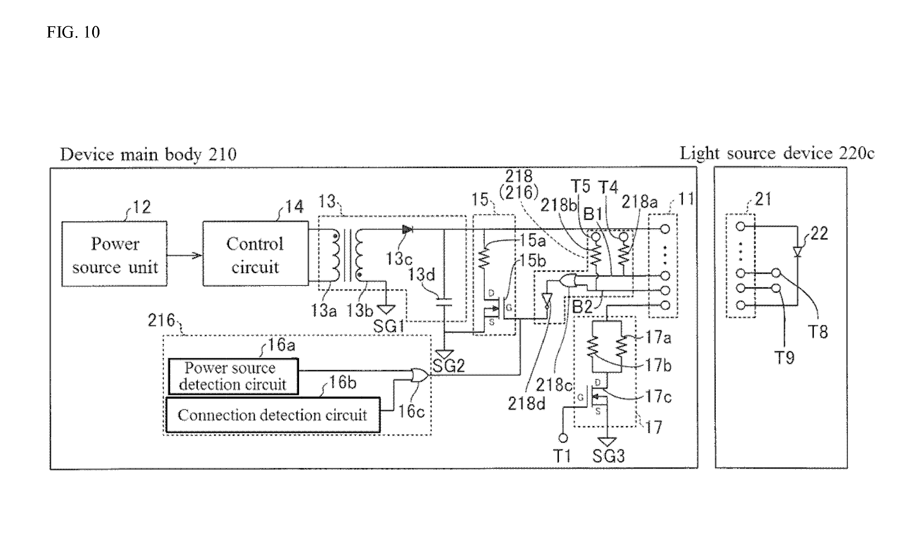

The threshold voltage is set to a value (8V) near a steady-state value (refer to FIG. 3, 10V) of the voltage applied to the FET 17c. As a result, from when the device main body 10 starts up until the threshold voltage is reached, the power source detection circuit 16a detects that the power source is off. Because the power is off during this time, such configuration allows the discharge circuit 15 to perform the discharge operation without a boosting operation being performed by the booster circuit 13. Therefore, from when the device main body 10 starts up until the threshold voltage is reached (in other words, until the device main body 10 stably rises), it may be possible to suppress malfunction occurring due to a booster operation being performed by the booster circuit 13.

The connection detection circuit 16b detects the connection of the light source device 20 to the main body connector 11, and the disconnection. The power source detection circuit 16b outputs a low level signal to the input end of the OR circuit 16c when it is detected that there is a connection, and output a high level signal to the input end of the OR circuit 16c when it is determined that the connection is removed.

The OR circuit 16 outputs a high or low level signal from the output end to the gate of the FET 15b of the discharge circuit 15 according to a signal from the power source detection circuit 16a and the connection detection circuit 16b.

According to one or more embodiments, the device main body 10 may comprise a current limiting circuit 17. The current limiting device 17 includes two resistances 17a and 17b, and an ET17c.

The two resistances 17a and 17b are connected in parallel with each other. The two resistances 17a and 17b have each of one end connected to a terminal of the main body side connector 11 and each of the other end connected to the drain of the FET 17c.

The FET 17c has a source connected to an SG3 and a gate connected to a terminal T1. In the terminal T1, an input voltage for turning the FET 17c on is input from the power source unit 12.

In the current limiting circuit 17, the device main body is started up, and the FET 17c is turned on when the input voltage from the power source unit 12 has reached a steady-state value (refer to FIG. 3, 10V). As a result, an LED 22 described later of the light source device 20 is connected to the SG3 via the light source side connector 21, the main body side connector 11, and the current limiting circuit 17 (the two resistances 17a and 17b, and the FET 17c). As a result, current flows to the LED by voltage applied by the booster circuit 13, and light is irradiated from the light source device 20 to the subject.

According to one or more embodiments, the light source device 20 may comprise an LED 22. The LED 22 is a plurality of LED elements being connected in series. The LED 22 has both ends each connected to a separate terminal of the light side connector 21, and is configured so that voltage is applied by the booster circuit 13 of the device main body 10 via the light source side connector 21.

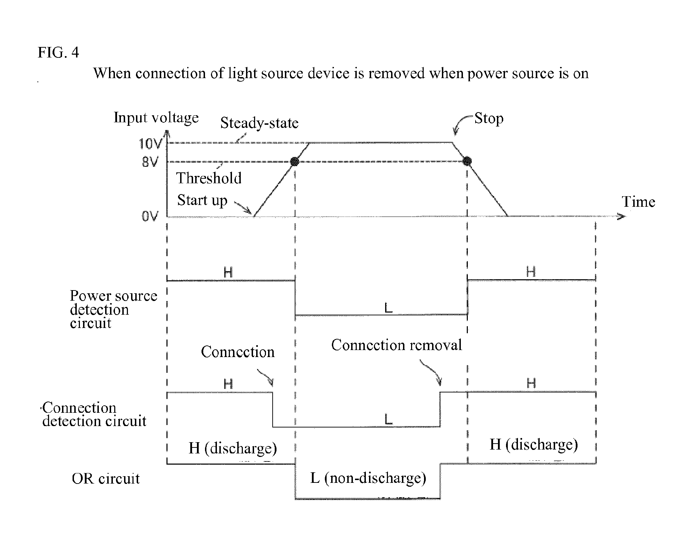

A control operation of the discharge circuit 15 by the discharge circuit controller 16 will be described with reference to FIGS. 3 and 4. First, a description will be given with reference to FIG. 3 for when the power source (power source unit 12) is off, and the connection between the light source device 20 and the main body side connector 11 is removed. It will be described step-by-step from the startup of the photoacoustic imaging device 100 until the connection between the light source device 20 and the main body side connector 11 is removed.

As illustrated in FIG. 3, while a user does not use the photoacoustic imaging device 100, the device main body 10 has the power source turned off, and the light source device 20 is removed from the main body side connector 11. In this case, the power source detection circuit 16a of the discharge circuit controller 16 detects that the power source is off. Then, a high level signal to the input end of the OR circuit 16c from the power source detection circuit 16a. The connection detection circuit 16b of the discharge circuit controller 16 detects that the connection of the light source device 20 and the main body side connector 11 is removed. Then, a high level signal is then output to the input end of the OR circuit 16c from the connection detection circuit 16b. Because a high level signal is input from both the power source detection circuit 16a and the connection detection circuit 16b, a high level signal is output to the FET 15b of the discharge circuit 15 from the OR circuit 16c as a result of a logic operation. As a result, the FET 15b turns on, and the discharge circuit 15 performs a discharge process.

The light source device 20 is connected to the main body side connector 11 by the user to use the photoacoustic imaging device 100. Then, when the device main body 10 is started up by the user, a supplying of power is started to each component of the device main body 10 such as the booster circuit 13 and a current return circuit 17 of the FET 17c. In FIG. 3, a change is illustrated according to the time of the input voltage applied to the FET 17c. As illustrated in FIG. 3, the input voltage applied to the FET 17c gradually increases when the device main body 10 starts up. The connection of the light source device 20 to the main body side connector 11 is not limited to before the startup of the device main body 10, and may be performed at any timing.

The power source detection circuit 16a detects that the power source is off until the input voltage exceeds the threshold voltage (8V). Then, a high level signal is output to the input end of the OR circuit 16c from the power source detection circuit 16a. Meanwhile, because the light source device 20 and the main body side connector 11 are connected, it is determined that the light source device 20 and the main body side connector 11 are connected in the connection detection circuit 16b. A low level signal is output to the input end of the OR circuit 16c from the connection detection circuit 16b. Because a high level signal is input from the power source detection circuit 16a and a low level signal is input from the connection detection circuit 16b, a high level signal is output to the FET 15b of the discharge circuit 15 from the OR circuit 16c as a result of a logic operation. As a result, the FET 15b is maintained on until the input voltage exceeds the threshold voltage (8V). In other words, even if the device main body 10 is started up, the booster circuit 13 does not perform the boosting operation until the input voltage exceeds the threshold voltage because the discharge operation is performed by the discharge circuit 15.

The power source detection circuit 16a then detects that the power source is on when the input voltage exceeds the threshold voltage (8V). Then, a low level signal is output to the input end of the OR circuit 16c from the power source detection circuit 16a. In this case, because a low level signal is input from both the power source detection circuit 16a and the connection detection circuit 16b, a low level signal is output to the FET 15b of the discharge circuit 15 from the OR circuit 16c as a result of a logic operation. As a result, the FET 15b turns off, and the discharge circuit 15 stops the discharge operation (non-discharge). Therefore, the booster circuit performs the boosting operation, and voltage is applied to the light source device 20.

When use of the photoacoustic imaging device 100 is completed and the power source of the device main body 10 is turned off by the user, the supply of electric power to each component of the device main body 10 from the power source unit 12 is stopped. At this time, as with when starting up, the input voltage applied to the FET 17c gradually decreases.

The power source detection circuit 16a detects that the power source is off when the input voltage is under the threshold voltage (8V). Then, a high level signal is output to the input end of the OR circuit 16c from the power source detection circuit 16a. Meanwhile, it is detected that the light source device 20 and the main body side connector 11 are connected in the connection detection circuit 16b. A low level signal is output to the input end of the OR circuit 16c from the connection detection circuit 16b. Because a high level signal is input from the power source detection circuit 16a and a low level signal is input from the connection detection circuit 16b, a high level signal is output to the FET 15b of the discharge circuit 15 from the OR circuit 16c as a result of a logic operation. As a result, the FET 15b turns on, and the discharge circuit 15 performs the discharge process. As a result, the discharge circuit rapidly discharges the charge stored in the capacitor 13d of the booster circuit 13 when the power source of the device main body 10 is turned off.

Then, the connection of the light source device 20 to the main body side connector 11 is removed. In this case, the connection detection circuit 16b detects that the connection of the light source device 20 to the main body side connector 11 is removed. A high level signal is output to the input end of the OR circuit 16c from the connection detection circuit 16b. Because a high level signal is input from both the power source detection circuit 16a and the connection detection circuit 16b, a high level signal is output to the FET 15b of the discharge circuit 15 from the OR circuit 16c as a result of a logic operation. As a result, the FET 15b is maintained on.

Next, an example will be described when the power source (power source unit 12) is on, and the connection of the light source device 20 and the main body side connector 11 is removed, with reference to FIG. 4. A description of details that are the same as those described referring to FIG. 3 will be omitted, and differences (the disconnection of the light source device 20 to the main body side connector 11 when the power source is on).

As illustrated in FIG. 4, the power source detection circuit 16a detects that the power source is on when the connection of the light source device 20 to the connector 11 is removed when the power source is on (when the input voltage exceeds the threshold voltage). Then, a low level signal is output to the input end of the OR circuit 16c from the power source detection circuit 16a. Moreover, the connection detection circuit 16b detects that the connection of the light source device 20 to the main body side connector 11 is removed. A high level signal is output to the input end of the OR circuit 16c from the connection detection circuit 16a. Because a low level signal is input from the power source detection circuit 16a and a high level signal is input from the connection detection circuit 16b, a high level signal is output to the FET 15b of the discharge circuit 15 from the OR circuit 16c as a result of a logic operation. As a result, the FET 15b turns on, and the discharge circuit 15 performs a discharge process. Therefore, the discharge circuit discharges the charge stored in the capacitor 13d of the booster circuit 13 when the connection of the light source device 20 to the main body side connector 11 is removed, even while on.

Embodiments of the first example of the present invention can achieve one or more effects below.

According to one or more embodiments of the first example of the present invention, as described above, the device main body 10 may comprise the discharge circuit controller 16 that controls the discharge circuit 15 to perform the discharge operation that discharges the charge stored in the capacitor 13d of the device main body 10 when the connection between the main body side connector 11 and the light source device 20 is removed and when the power source of the device main body 10 is off. As a result, the discharge circuit 15 may discharge the charge stored in the capacitor 13d of the device main body 10 even when the power source (power source unit 12) is on when the connection between the main body side connector 11 and the light source device 20 is removed. The discharge circuit 15 rapidly discharges the charge stored in the capacitor 13d of the device main body 10 when the power source is off. As a result, damage of an electric circuit can be suppressed due to the charge stored in the capacitor 13d in the device main body 10 in both cases when the connection between the main body side connector 11 and the light source device 20 is removed or when the power source of the device main body 10 is off.

According to one or embodiments of the first example of the present invention, the discharge circuit controller 16 may comprise the power source detection circuit 16a that detects whether a power source is on or off, and the connection detection circuit 16b that detects the connection of the light source device 20 to the main body connector 11, and the disconnection. As a result, along with being able to simply detect whether a power source is on or off by the power source detection circuit 16a, the connection detection circuit 16b can also simply detects whether there is a connection or a connection has been removed.

According to one or embodiments of the first example of the present invention, as described above, the power source detection circuit 16a determines whether the power source is on when it is detected that the voltage of the power source (voltage applied to the gate of the FET 17c of the current limiting circuit 17 from the power source unit 12) exceeds a threshold voltage, and to determine that the power source is off when the threshold voltage is not exceeded. As a result, whether a power source is on or off can be easily determined by only detecting that the voltage of the power source has exceeded a threshold voltage.

Moreover, according to the first example, as described above, the discharge circuit 15 includes the FET 15b that performs a switch of the discharge operation by turning on and off according to a signal output from the discharge circuit controller 16. As a result configuration, the discharge operation of the discharge circuit 15 can be switched with a simple configuration.

### (Second Example)

Embodiments of the second example of the present invention will be described with reference to FIGS. 1,5, and 6. Embodiments of the second example of the present invention will be described where the discharge operation by the discharge circuit 15 is performed when it has been detected that a non-genuine product of a light source device 120 has been connected, in addition to the configuration of embodiments of the first example of the present invention.

A photo acoustic imaging device 200 according to one or more embodiments of the second example of the present invention may comprise a photoacoustic imaging device main body 110 (disclosed as device main body 110 below), and a light source device 120, as illustrated in FIG. 1. Configurations that are the same as that in embodiments of the first example will be shown with the same numerals and descriptions thereof are omitted.

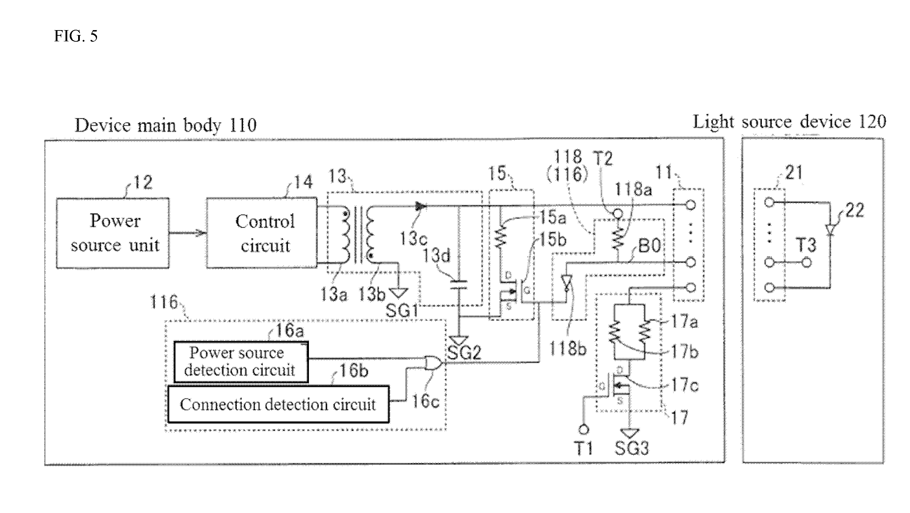

According to one or more embodiments of the second example of the present invention, as illustrated in FIG. 5, the device main body 110 may comprise a discharge circuit controller 116. According to one or more embodiments, the discharge circuit controller 116 includes a genuine product detection circuit 118 in addition to the power source detection circuit 16a, the connection detection circuit 16b. The genuine product detection circuit 118 is an example of a "genuine product detector" of one or more embodiments of the present invention.

According to one or more embodiments of the second example of the present invention, the genuine product detection circuit 118 detects that either a genuine product or a non-genuine product of the light source device 120 has been connected. The discharge circuit controller 116 controls the discharge circuit 15 to perform the discharge operation when the genuine product detection circuit 118 detects that a non-genuine product has been connected, in addition to when the main body side connector 11 and the light source device 20 are disconnected, or when the power source (power source unit 12) of the device main body 10 is off. The discharge circuit controller 116 controls the discharge circuit 15 not to perform the discharge operation when the genuine product detection circuit 118 has detected that a genuine product has been connected, and when the power source of the device main body 110 is on.

According to one or more embodiments, the genuine product detector 118 includes a pull up resistance 118a, and an NOT circuit 118b.

The pull up resistance 118a includes one end connected to a terminal T2. A pull up voltage (voltage corresponding to a high level signal) applied to the pull up resistance 118a is input into the terminal T2. The pull up resistance 118a has the other end connected to an input end of the NOT circuit 118 and a terminal of the main body side connector 11.

The NOT circuit 118b includes the input end connected to the terminal of the main body side connector 11 and the pull up resistance 118a and the output end connected to the gate of the FET 15 of the discharge circuit 15.

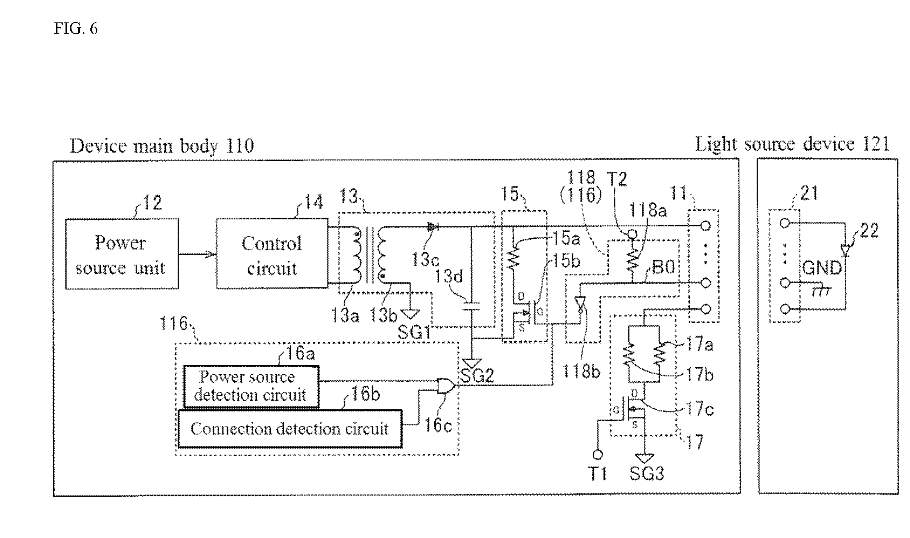

Next, a control operation of the discharge circuit 15 according to the detection of a genuine product or a non-genuine product by the genuine product detection circuit 118 (discharge circuit controller 116) will be described with reference to FIGS. 5 and 6.

As illustrated in FIG. 5, the pull up resistance 118a is connected to a terminal T3 of the light source device 120 side via the main body side connector 11 when a genuine product of the light source device 120 is connected to the main body side connector 11. In this case, a high level voltage is maintained in a wiring B0 of the genuine product detection circuit 118. As a result, a signal of high level voltage is input into the input end of the NOT circuit 118b. The NOT circuit 118b inverts the high level voltage. As a result, a signal of low level voltage is output from the NOT circuit 118b to the gate of the FET 15b. Then, a low level signal is output from the OR circuit 16c and the FET 15b is turned off in the same manner as the first example when the power source of the device main body 110 is turned on with the genuine product of the light source device 120 connected. As a result, the discharge circuit does not perform the discharge operation.

As illustrated in FIG. 6, the pull up resistance 118a is connected to a GND of a light source device 121 side via the main body side connector 11 when a non-genuine product (defined here as the light source device 121) of the light source device 120 is connected to the main body side connector 11. In this case, a lowering of voltage occurs in the wiring B0 of the genuine product detection circuit 118, and becomes a low level voltage. As a result, a signal of low level voltage is input into the input end of the NOT circuit 118b. The NOT circuit 118b inverts low level voltage signal. As a result, a signal of high level voltage is output from the NOT circuit 118b to the gate of the FET 15b. As a result, the FET 15b is turned on, and a discharge process is performed by the discharge circuit 15. A non-genuine product of the light source device 120 can be detected if a lowering of voltage occurs in the wiring B0 even when the pull up resistance 118a is connected to anything other than the GND of the light source device 121 side.

As described above, according to one or more embodiments of the second example of the present invention, the genuine product detection circuit 118 (discharge circuit controller 116) can detect a genuine product or a non-genuine product and control the discharge operation based on the detection results.

The other configurations of embodiments of the second example of the present invention are the same as that of embodiments of the first example of the present invention.

Embodiments of the second example of the present invention can achieve one or more effects below.

According to one or more embodiments of the second example of the present invention, as described above, the device main body 110 may comprise the discharge circuit controller 116 that controls the discharge circuit 15 to perform the discharge operation when the main body side connector 11 and the light source device 120 are disconnected and when the power source (power source unit 12) of the photoacoustic imaging device main body 110 is off. As a result, as with embodiments of the first example of the present invention, the protection of an electrical circuit can be surely performed by discharging a charge of the capacitor 13d of the device main body 110 when the power source is off.

According to one or more embodiments of the second example of the present invention, as described above, the discharge circuit controller 116 may comprise the genuine product detection circuit 118 that detects that either a genuine product or a non-genuine product (light source device 121) of the light source device 120 has been connected. Then, the discharge circuit controller 116 controls the discharge circuit 15 to perform the discharge operation when the genuine product detection circuit 118 has detects that a non-genuine product has been connected in addition to when the main body side connector 11 and the light source device 20 are disconnected, or when the power source is off. As a result, the booster circuit 13 may not perform the boosting operation because the discharge circuit 15 performs the discharge operation when a non-genuine product of the light source device 120 is accidentally connected. Therefore, the application of voltage that has been boosted by the booster circuit 13 to a non-genuine product can be suppressed, even if a non-genuine product (light source device 121) of the light source device 120 is accidentally connected.

According to one or more embodiments of the second example of the present invention, as described above, the discharge circuit controller 116 controls the discharge circuit 15 not to perform the discharge operation when the genuine product detection circuit 118 has detected that a genuine product has been connected, and when on. As a result, the booster circuit 13 may appropriately perform the boosting operation when a genuine product of the light source device 120 is connected.

The other effects of embodiments of the second example of the present invention may be the same as that of embodiments of the first example of the present invention.

### (Third Example)

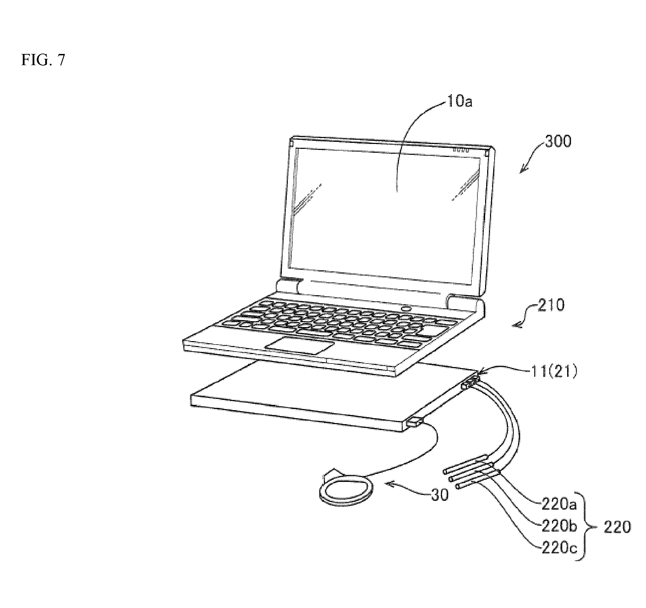

Embodiments of the third example of the present invention will be described with reference to FIGS. 7 through 11. According to one or more embodiments of the second example of the present invention, the photoacoustic imaging device 200 including one light source device 120 detects the genuine product or the non-genuine product. On the other hand, according to one or more embodiments of the third example of the present invention, a photoacoustic imaging device 300 including a plurality (three) of light source devices 220 (220a, 220b, and 220c) detects a genuine product or a non-genuine product.

A photo acoustic imaging device 300 according to one or more embodiments of the third example of the present invention may comprise a photoacoustic imaging device main body 210 (disclosed as device main body 210 below), and three light source devices 220 (220a through 220c). In the photoacoustic imaging device 300, the three light source devices 220 (220a through 220c) are each configured to generate light of different wavelengths. According to the photoacoustic imaging device 300, information inside of a subject can be obtained in more detail by using light of different wavelengths. Configurations of embodiments of the third example of the present invention that are the same as those of embodiments of the first and second examples of the present invention will be shown with the same numerals and descriptions thereof are omitted.

According to one or more embodiments, as illustrated in FIG. 8, the device main body 210 may comprise a discharge circuit controller 216. According to embodiments of the third example of the present invention, the discharge circuit controller 216 includes a genuine product detection circuit 218 in addition to the power source detection circuit 16a, the connection detection circuit 16b. The genuine product detection circuit 218 is one example of a "genuine product detector" of one or more embodiments of the present invention.

The genuine product detection circuit 218 detects that either a genuine product or a non-genuine product of the three light source devices 220 (220a through 220c) have been connected as with embodiments of the second example of the present invention. The discharge circuit controller 216 controls the discharge circuit 15 to perform the discharge operation when the genuine product detection circuit 218 has detected that a non-genuine product has been connected, in addition to when the main body side connector 11 and the light source device 220 are disconnected, or when the power source (power source unit 12) of the device main body 210 is off. The discharge circuit controller 216 controls the discharge circuit 15 not to perform the discharge operation when the genuine product detection circuit 218 has detected that a genuine product has been connected, and when the device main body 210 is on.

According to one or more embodiments, the genuine product detection circuit 218 may comprise two pull up resistances 218a and 218b, and OR circuit 218c, and an NOT circuit 218d.

The two pull up resistances 218a and 218b each includes one end connected to terminals T4 and T5. A pull up voltage (voltage corresponding to a high level signal) that applies to the pull up resistances 218a and 218b are input into each of the terminals T4 and T5. The two pull up resistances 218a and 218b includes the other ends connected to the input end of the OR circuit 218c. The other ends of the pull up resistances 218a and 218b are each connected to separate terminals of the main body side connector 11.

The output end of the OR circuit 218c is connected to the input end of the FET 218d. The output end of the NOT circuit 218d is connected to the gate of the FET 15b.

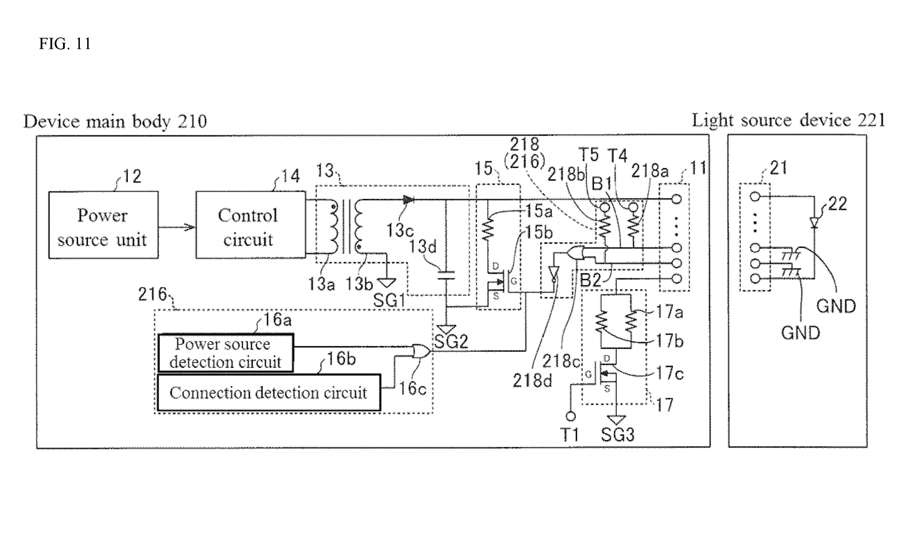

A control operation of the discharge circuit 15 according to the detection of a genuine product or a non-genuine product by the genuine product detection circuit 218 (discharge circuit controller 216) will be described with reference to FIGS. 8 through 11. FIGS. 8 through 10 illustrate when a genuine product (light source device 220a to 220c) of the light source device 220 is connected, and FIG. 11 illustrates when a non-genuine product (in this case, for example, a light source device 221) of the light source device 220 is connected.

As illustrated in FIG. 8, the pull up resistance 218a is connected to a terminal T6 of the light source device 220a side via the main body side connector 11 when a genuine product (light source device 220a) of the light source device 220 is connected to the main body side connector 11. The pull up resistance 218b is connected to the GND of the light source device 220a side. In this case, a high level voltage is maintained in a wiring B1 of the genuine product detection circuit 218. Meanwhile, a lowering of voltage occurs in a wiring B2 of the genuine product detection circuit 218, and becomes a low level voltage. As a result, a signal of high and low level voltage is input into the input end of the OR circuit 218c. Therefore, a high level signal is output to the NOT circuit 218d from the output end of the OR circuit 218c. The NOT circuit 218d inverts the high level voltage signal. As a result, a signal of low level voltage is output from the NOT circuit 218d to the gate of the FET 15b. Then, a low level signal is output from the OR circuit 16c and the FET 15b is turned off in the same manner as the first and second examples when the power source of the device main body 210 is turned on with a genuine product (light source device 220a) of the light source device 220 connected. As a result, the discharge circuit may not perform the discharge operation.

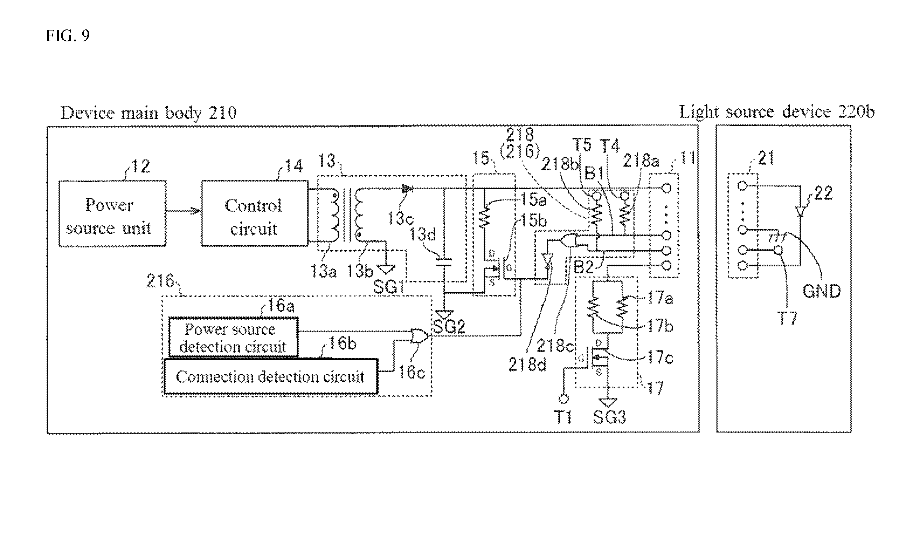

As illustrated in FIG. 9, the pull up resistance 218a is connected to the GND of the light source device 220b side via the main body side connector 11 when a genuine product (light source device 220b) of the light source device 220 is connected to the main body side connector 11. The pull up resistance 218b is connected to the terminal of the light source device 220b side. In this case, a lowering of voltage occurs in the wiring B1 of the genuine product detection circuit 218, and becomes a low level voltage. Meanwhile, a high level voltage is maintained in the wiring B2 of the genuine product detection circuit 218. As a result, a signal of low and high level voltage is input into the input end of the OR circuit 218c. Therefore, a high level signal is output to the NOT circuit 218d from the output end of the OR circuit 218c. The NOT circuit 218d inverts the high level voltage signal. As a result, a signal of low level voltage is output from the NOT circuit 218d to the gate of the FET 15b. Therefore, the discharge circuit does not perform the discharge operation as with when the light source device 220a is connected.

As illustrated in FIG. 10, the pull up resistance 218a and the pull up resistance 218b are each connected to the terminal T8 and terminal T9 of the light source device 220c side via the main body side connector 11 when a genuine product (light source device 220c) of the light source device 220 is connected to the main body side connector 11. In this case, a high level voltage is maintained in both wirings B1 and B2 of the genuine product detection circuit 218. As a result, a signal of high level voltage is input into the input end of the OR circuit 218c from the wirings B1 and B2. Therefore, a high level signal is output to the NOT circuit 218d from the output end of the OR circuit 218c. The NOT circuit 218d inverts the high level voltage signal. As a result, a signal of low level voltage is output from the NOT circuit 218d to the gate of the FET 15b. Therefore, the discharge circuit may not perform the discharge operation as with when the light source device 220a and the light source 220b are connected.

As illustrated in FIG. 11, the pull up resistance 218a and the pull up resistance 218b are each connected to GND and GND of the light source device 221 side via the main body side connector 11 when a genuine product (light source device 221) of the light source device 220 is connected to the main body side connector 11. In this case, a lowering of voltage occurs in the wirings B1 and B2 of the genuine product detection circuit 218, and becomes a low level voltage. As a result, a signal of low level voltage is input into the OR circuit 218c from the wirings B1 and B2. Therefore, a low level signal is output to the NOT circuit 218d from the output end of the OR circuit 218c. The NOT circuit 218d inverts the low level voltage signal. Thus, a signal of high level voltage is inverted and then the high level voltage signal is output from the NOT circuit 218d to the gate of the FET 15b. As a result, the FET 15b is turned on and the discharge circuit performs the discharge operation when it has been detected that a non-genuine product (light source device 221) of the light source device 220 has been connected. A non-genuine product of the light source device 220 can be detected if a lowering of voltage occurs in the wirings B1 and B2 even when the pull up resistances 218a and 218b are connected to anything other than the GND of the light source device 221 side.

According to one or more embodiments, as described above, it may be possible to detect a genuine product or a non-genuine product by a genuine product detection circuit and control the discharge operation based on the detection results when the photoacoustic imaging device 300 may comprise a plurality (three) of light source devices 220 (220a through 220c) by performing a logic operation using signals of the wirings B1 and B2.

The other configurations of embodiments of the third example of the present invention may be the same as that of embodiments of the first example of the present invention.

Embodiments of the third example of the present invention can achieve one or more effects below.

According to one or more embodiments of the third example of the present invention, as described above, the device main body 210 may comprise the discharge circuit controller 216 that controls the discharge circuit 15 to perform the discharge operation when the main body side connector 11 and the light source device 220 are disconnected or when the power source (power source unit 12) of the device main body 210 is off. As a result, as with embodiments of the first and second examples of the present invention, the protection of an electrical circuit can be surely performed by discharging a charge of the capacitor 13d of the device main body 210 when the power source is off.

According to one or more embodiments of the third example of the present invention, by configuring the genuine product detection circuit 218 as described above, it can be surely detected that either a genuine product or a non-genuine product of the light source device 220 is connected when the photoacoustic imaging device 300 comprises a plurality (three) of light source devices 220 (220a through 220c).

The other effects of embodiments of the third example of the present invention may be the same as that of embodiments of the first example of the present invention.

The present invention is not limited to embodiments described above. One or more embodiments of the present invention may include one or more of the following:

For example, according to one or more embodiments of the first through third examples of the present invention, the FET 15b is an example of the switching element of the discharge circuit 15, but the present invention is not limited to this. One or more embodiments of the present invention may use something other than and FET as the switching element. For example, the switching element may be a bipolar transistor.

According to one or more embodiments of another example of the present invention, the light emitting element of the light source device 20 (120, 220) may be configured by a light emitting diode. According to this configuration, for example, irradiation range of light is not easily changed relatively even when positions of the light emitting element is misaligned because directivity of the light emitting diode is lower than that of a light emitting semiconductor element. As a result, enlargement and complication of the photoacoustic imaging device may be suppressed because precise alignment of optical components, an optical surface plate, and a strong case are not required.

According to one or more embodiments of another example of the present invention, the light emitting element of the light source device 20 (120, 220) may be configured by a semiconductor laser element. According to this configuration, for example, the photoacoustic imaging device 10 (110, 210) may cause the semiconductor laser element to surely irradiate a large part of light to a subject because directivity of the semiconductor laser element is higher than that of a light emitting diode.

According to one or more embodiments of another example of the present invention, the light emitting element of the light source device 20 (120, 220) may be configured by an organic light emitting diode. According to this configuration, for example, the photoacoustic imaging device 10 (110, 210) may cause the light source device 20 (120, 220) including light emitting semiconductor element to be easily downsized because the organic light emitting diode is easily thinned.

According to one or more embodiments of the first example of the present invention, the power source unit and connection detector are each configured by the power source detection circuit 16a and the connection detection circuit 16b. According to one or more embodiments of the second and third examples of the present invention, the genuine product detector is configured by the genuine product detection circuit 118 (218). According to one or more embodiments of the present invention, the power source detector, connection detector, and genuine product detector may be configured by a microcomputer or a CPU.

According to one or more embodiments of the first example of the present invention, the discharge circuit controller 16 may comprise the power source detection circuit 16a, the connection detection circuit 16b, and the OR circuit 16c. According to one or more embodiments of the second and third examples of the present invention, the discharge circuit controller 116 (216) may comprise the genuine product detection circuit 118 (218). However the present invention is not limited to this. One or more embodiments of the present invention may configure the discharge circuit controller by a microcomputer or a CPU, and the microcomputer or CPU as the discharge circuit controller may comprise the functions described above.

According to one or more embodiments of the first example, the threshold voltage may be set to 8V, but the present invention is not limited to this. A voltage other than 8V may be set as the threshold voltage in one or more embodiments of the present invention.

According to one or more embodiments of the second example of the present invention, it may be detected that either a genuine product or a non-genuine product of one light source device 120 is connected. According to one or more embodiments of the third example of the present invention, it may be detected that either a genuine product or a non-genuine product of three light source devices 220 (220a through 220c) is connected. However, the present invention is not limited to this. The number of light source devices may be plural such as two or four or greater.

Although the disclosure has been described with respect to only a limited number of embodiments, those skilled in the art, having benefit of this disclosure, will appreciate that various other embodiments may be devised without departing from the scope of the present invention. Accordingly, the scope of the invention should be limited only by the attached claims.

### [Explanation of References]

10, 110, 210 Photoacoustic imaging device main body
11 Main body side connector (connector)
13 Booster circuit
13d Capacitor
15 Discharge circuit
16, 116, 216 Discharge circuit controller
16a Power source detection circuit (power source detector)
16b Connection detection circuit (connection detector)
20, 120, 220 Light source device
100, 200, 300 Photoacoustic imaging device
118,218 Genuine product detection circuit (genuine product detector)

## Claims

1. A photoacoustic imaging device comprising:
a connector (11) that connects a light source device (20, 120, 220);
a booster circuit (13) that includes a capacitor (13d) and that performs a boosting operation that applies voltage to the light source device via the connector;
a discharge circuit (15) connected to both poles of the capacitor ofthe booster circuit and that performs a discharge operation that discharges a charge stored in the capacitor (13d); and
a discharge circuit controller (16, 116, 216) that controls the discharge circuit to perform the discharge operation when the connector (11) and the light source device (20) are disconnected or when the power source of the photoacoustic imaging device main body is off.

2. The photoacoustic imaging device according to claim 1, wherein the discharge circuit controller comprises a power source detector (16a) that detects whether a power source is on or off.

3. The photoacoustic imaging device according to claim 1 or 2, wherein the discharge controller comprises a connection detector (16b) that detects whether the light source device and the connector are connected or disconnected.

4. The photoacoustic imaging device according to any one of claims 1 to 3, wherein the discharge circuit controller comprises a switching element (15b) and switches the discharge operation by switching the switching element on and off according to a signal output from the discharge circuit controller.

5. The photoacoustic imaging device according to any one of claims 1 to 3, wherein
the discharge circuit controller (116)
comprises a genuine product detector (118) that detects whether a genuine product or a non-genuine product (120) of the light source device is connected, and
controls the discharge circuit to perform the discharge operation when the genuine product detector (115) detects that a non-genuine product is connected.

6. The photoacoustic imaging device according to claim 5, wherein the discharge circuit controller (116) controls the discharge circuit not to perform the discharge operation when the genuine product detector detects that a genuine product is connected and when the power source (12) is on.

7. The photoacoustic imaging device according to claim 2, wherein
the power source detector (16a)
determines that the power source is on when a voltage higher than a predetermined threshold voltage is detected, and
determines that the power source is off when a voltage lower than a predetermined threshold voltage is detected.

8. The photoacoustic imaging device according to any one of claims 1 to 7, wherein the booster circuit (13) stops the boosting operation while performing the discharge operation.

9. The photoacoustic imaging device according to claim 4, wherein the discharge circuit controller further comprises a discharge resistance (15a).

10. The photoacoustic imaging device according to claim 9, wherein
the booster circuit (13) stops the boosting operation for a time period longer than a time constant determined based on the discharge resistance (15a) and the capacitor (13d) of the booster circuit.

11. The photoacoustic imaging device according to any one of claims 1 to 10, wherein a light emitting element of the light source device comprises a plurality of light emitting elements connected in series.

12. The photoacoustic imaging device according to any one of claims 1 to 11, wherein a light emitting element of the light source device is a light emitting diode (22).

13. The photoacoustic imaging device according to any one of claims 1 to 11, wherein a light emitting element of the light source device is a semiconductor laser element.

14. The photoacoustic imaging device according to any one of claims 1 to 11, wherein a light emitting element of the light source device is an organic light emitting diode.
